Europäisches Patentamt

European Patent Office

Office européen des brevets

(19)

(11) Publication number: **0 364 887**
**A2**

## EUROPEAN PATENT APPLICATION

(12)

(21) Application number: **89119011.8**

(22) Date of filing: **12.10.89**

(51) Int. Cl.⁵: **A61K 7/06 , A61K 7/11**

(30) Priority: **19.10.88 US 259832**

(43) Date of publication of application:
**25.04.90 Bulletin 90/17**

(84) Designated Contracting States:
**BE DE FR GB IT NL SE**

(71) Applicant: **National Starch and Chemical Corporation**
**10 Finderne Avenue, P.O. Box 6500**
**Bridgewater, New Jersey 08807(US)**

(72) Inventor: **Morawsky, Natalie**
**26C Cedar Lane**
**Highland Park New Jersey 08904(US)**
Inventor: **Gallaway, George**
**RD No. 162 West Portal**
**Asbury New Jersey 08802(US)**
Inventor: **Sanders, Michael**
**124 Featherbed Lane**
**Flemington New Jersey 08822(US)**

(74) Representative: **Hagemann, Heinrich, Dr. et al**
**Patentanwälte Hagemann & Kehl Postfach**
**860329**
**D-8000 München 86(DE)**

(54) High hydrocarbon tolerant hair fixing compositions having superior shampoo removability.

(57) This invention presents novel hair fixing compositions comprising copolymers of acrylates or methacrylates, N-substituted acrylamides and acrylic acid. Such materials, when neutralized by an appropriate neutralizing agent exhibit an enhanced tolerance of non-halogenated hydrocarbons, yet also exhibit a high degree of shampoo removability, making them particularly useful in aerosol formulations using non-halogenated hydrocarbon propellants.

EP 0 364 887 A2

# HIGH HYDROCARBON TOLERANT HAIR FIXING COMPOSITIONS HAVING SUPERIOR SHAMPOO REMOVABILITY

## 1. BACKGROUND OF THE INVENTION

This invention relates to novel hair fixing compositions which exhibit superior hydrocarbon tolerance, and are thus suitable for use in aerosol applications wherein hydrocarbon propellants are used, and which also are easily removed from the hair by shampooing.

In order to be effective in aerosol hair spray formulations, the film forming, polymeric binders utilized therein as well as the films derived therefrom must meet a rigid set of requirements. The binders used in such formulations should be soluble in organic solvents and completely compatible with the propellants and solvents ordinarily employed in the aerosols; yet the films cast on the hair from such formulations should, ordinarily, be either water soluble or water dispersible in order to facilitate their easy removal from the user's hair by shampooing. As is readily visualized, this is an unusual combination of properties which is further complicated by the requirement that the binder used in such formulations be stable in the presence of, and unreactive with, the perfumes or other optional ingredients utilized in hair spray formulations.

Further, the films cast from the solutions of these binders should be flexible and yet they should have sufficient strength and elasticity to hold the hair; they should adhere well to hair so as to avoid dusting or flaking off when the hair is subjected to varying stresses; they should readily allow the hair to be recombed; they should maintain a nontacky state despite varying environmental conditions; they should be clear, transparent and glossy and should maintain this clarity on aging; they should possess good anti-static properties; and, as previously noted, they should be easily removable by the use of water and/or soap or shampoo.

Many polymeric systems have been utilized in an attempt to meet these stringent requirements. Among these are included; polyvinylpyrrolidone, copolymers of N-vinyl pyrrolidone with vinylacetate, 5-5'-dimethyl hydantoinformaldehyde resins and copolymers of methyl vinyl ethers and maleic acid half esters, etc. Though each of the latter systems has met at least some of the above cited requirements, none has exhibited all of these requirements to an optimum degree.

For example, carboxylated vinyl polymeric hair spray resins, particularly the carboxylated acrylate, and/or acetate based resins, have long been favored for use in aerosol hair spray formulations. Also useful are a class of carboxylated ester polymers comprising an acrylamide, an acidic film forming comonomer, and at least one polymerizable comonomer which are described in U.S. Pat. No. 3,927,199. In order to obtain optimum benefits for the use of such acidic resins, it has been required to neutralize at least a portion, and preferably most or all, of the available carboxyl functionalities with specific alkaline reagents, e.g., amines and aminohydroxy compounds, as described in, for example, U.S. Pat Nos. 2,996,471; 3,405,084; 3,577,517, etc. Thus, alkaline reagents which are suggested for such neutralizations include ammonia, lithium hydroxide, potassium hydroxide, sodium hydroxide, mono-, di- or triethanolamine, mono-, di- or tripropanolamine, morpholine, amino methyl propanol, amino methyl propanediol, hydroxy ethyl morpholine, and mixtures thereof. The purpose of this neutralization step is both to improve the water solubility or dispersibility of the resin thus permitting easy removal from the hair by merely washing with shampoo and also to affect the degree of flexibility of the resultant film when sprayed on the hair (i.e., to produce a soft film, normal film or a film suitable for "hard to hold" hair). Additionally, United States Pat. No. 4,192,861 teaches the use of long chain amines for the neutralization of specific polymers in aerosol hairspray systems.

One class of neutralizing agent which has been extensively used in the hair spray industry is the amines, especially 2-amino-2-methyl-1-propanol (AMP) and 2-amino-2- methyl-1,3 propanediol (AMPD). These agents are quite versatile in their utility and are used in a number of formulations marketed by a number of manufacturers.

Until recently, the majority of aerosol formulations employed halogenated hydrocarbons, particularly the chlorofluorocarbons as propellants. Such propellants were uniquely suitable for use in aerosol systems since they were compatible with polar solvents, including water. The resins employed can also have high solubility in these solvents and, thus, can be easily removed from the hair by shampooing.

Recent ecological concerns, however, have resulted in a shift away from the use of halogenated hydrocarbon propellants and cosolvents and toward the use of hydrocarbons as propellants in aerosol hair spray formulations. In such systems, the binder and any optional ingredients are dissolved in a suitable solvent, such as an alcohol, and the hydrocarbon serves as the propellant. Unfortunately, the use of these propellants produces a number of problems, some of which are due to the decrease in solubility of the binder in the solvent system as the hydrocarbon content is increased. Thus, while the carboxylated resins are soluble in the anhydrous alcohol-halocarbon systems of the prior art, and are the commercially preferred resins for their hair holding properties, their reduced solubility in the alcohol-hydrocarbon propellant may render them unacceptable to the industry for use in aerosol systems containing high levels of hydrocarbon propellants.

A number of hydrocarbon tolerant carboxylated resins have, thus, been developed for use with hydrocarbon propellants. The development, however, presents a double-edged sword, for once the systems become sufficiently tolerant of the non-polar propellant, they ordinarily become extremely resistant to water dispersion and thus, to the removal from hair by shampooing. While this resistance to shampoo removal has been observed, to a greater or lesser extent, with all hydrocarbon tolerant carboxylated resins, some resins have been developed which lessen the problem.

One acrylate-based resin marketed by National Starch and Chemical Corporation exhibits exceptionally high hydrocarbon tolerance. The resin, a terpolymer comprising 30% (by weight) N-t-octylacrylamide, 51% isobutylmethacrylate, and 19% acrylic acid, has enjoyed wide acceptance by the industry. When neutralized by an appropriate neutralizing agent, the resin is tolerant to a high concentration of hydrocarbon and exhibits an acceptable level of shampoo removability. However, complete removal of the film from the hair can require repeated washes, making it unattractive in some applications.

Thus, there exists a need for hair spray formulations which exhibit acceptable shampoo removability as well as high tolerance of hydrocarbon propellants which are becoming increasingly important in the industry.

Accordingly, it is an object of this invention to present a series of hydrocarbon tolerant hair fixing compositions suitable for use in aerosol formulations with hydrocarbon propellants which are also shampoo removable.

## 2. SUMMARY OF INVENTION

This invention presents a series of novel hair fixing compositions meeting the above objects which comprise a class of copolymers of alkyl acrylates or methacrylates, N-substituted acrylamides, and acrylic acid. Such compositions, when neutralized with an appropriate neutralizing agent, exhibit a high tolerance to hydrocarbons, yet the films cast on the hair by such resins are easily removable by shampooing. Thus, the compositions are especially useful in aerosol formulations utilizing hydrocarbon propellants.

This unique combination of properties is achieved by copolymerizing about 0 - about 60% (by weight) of a $C_3$-$C_{12}$ alkyl acrylate or methacrylate, about 15 - about 75% (by weight) $C_4$-$C_{10}$ N-substituted acrylamide, and about 20 - about 35% (by weight) acrylic acid or methacrylic acid; other optional comonomers may also be included. These resultant copolymers can be neutralized by any convenient basic neutralizing agent, with the preferred class being the amines, preferably AMP or AMPD.

The neutralized copolymers, when subsequently employed in an aerosol system, will tolerate high levels of hydrocarbon propellant in the aerosol system, yet maintain a high degree of shampoo removability. Advantageously, this permits a much higher level of polymer to be present in the aerosol formulation.

## DETAILED DESCRIPTION OF THE INVENTION

## 3.1 POLYMERS USED IN THE HAIR SPRAY FORMULATIONS

## 3.1.1 ACRYLATE-BASED COPOLYMERS

One class of copolymers especially suited for use in hair spray formulations containing hydrocarbon propellants are copolymers containing about 0 - about 60% (by weight) of a $C_3$-$C_{12}$, preferably $C_3$ - $C_8$, alkyl acrylate or methacrylate, about 15 - about 75% (by weight) of a $C_4$ - $C_{10}$ N-substituted alkly acrylamide, and about 20 - about 35% acrylic acid or methacrylic acid, wherein the percentages total 100%. More preferably, these copolymers comprise about 30 - about 50% isobutylmethacrylate, about 25 - about 35% N-t-octyl acrylamide, and about 20 - about 30% acrylic acid. This copolymer is a solid which is soluble in alcohols and other materials which can be used as solvents used in hair spray formulations such as methanol, ethanol, isopropanol, and acetone as well as some other esters and ketones. Preferably, the

3

copolymers will possess intrinsic viscosities of 0.25 -0.45, but these values are not exclusive, and can be varied as the applications dictate.

These copolymers exhibit superior hair-holding properties and, when applied as an aerosol to the hair, produce a hard but flexible film which displays excellent subjective properties such as high gloss, good static resistance, and superior adhesion to hair.

Further, when neutralized by an appropriate neutralizing agent as described in Section 3.2, the copolymers are tolerant to hydrocarbons, permitting high levels of hydrocarbon propellants, often 50-60% (by weight) or more of the formulation, to be present. Yet the films formed by these resins when applied to hair exhibit a high degree of shampoo removability.

## 3.1.2 OPTIONAL COMONOMERS

In addition to the comonomers described in 3.1.1, the copolymers can also contain from about 2.5 to about 20% of one or more optional comonomers. These monomers can be included to tailor and/or enhance certain properties of the copolymer such as hair adherance, hardness, flexibility, antistatic properties and the like. Among these comonomers are hydroxyalkyl esters of acrylic and methacrylic acids such as hydroxypropyl acrylate and methacrylate, hydroxybutyl acrylate andmethacrylate, hydroxystearyl acrylate and methacrylate and hydroxyethyl acrylate and methacrylate; alkyl $(C_1-C_4)$ / amino alkyl $(C_2-C_4)$ esters of acrylic and methacrylic acids such as N,N-diethylaminoethyl acrylate, N-t-butylaminopropyl acrylate, N, N-dimethylaminoethyl methacrylate, N-t-butylaminoethyl methacrylate, and the quaternization product of dimethylaminoethyl methacrylate and dimethyl sulfate, diethyl sulfate and the like; diacetone acrylamide; and vinyl esters such as vinyl acetate and vinyl propionate; styrene and alkyl-substituted monomers such as styrene and alpha-methyl styrene; $C_1-C_8$ dialkyl maleates; N-vinyl pyrrolidone; and N-substituted alkyl $(C_1-C_8)$ maleamic acids.

## 3.2 NEUTRALIZATION

As stated supra, water solubility (and hence shampoo removability) of acidic polymer compositions used in hair sprays is achieved by neutralization with an appropriate alkaline material. Generally, this results in a decrease in hydrocarbon tolerance. However, the hydrocarbon tolerance of the copolymers of this invention remains quite high after neutralization, permitting high levels of hydrocarbon propellant to be present in the resultant hair spray formulation. Further, use of neutralizing agents which are soluble in alcohol permits the polymer compositions to be neutralized directly in the hair spray formulation, eliminating the need for an additional preparation step.

Many alkaline neutralizing agents may be employed including NaOH, KOH, and mixtures of NaOH and/or KOH with long chain amines such as those described in U.S. Pat. 4,192,861 issued March 11, 1980, to Micchelli et al., and incorporated herein by reference. However, the preferred neutralizing agents are organic amines, preferably 2-amino-2-methyl-1- propanol (AMP) and 2-amino-2-methyl-1,3-propanediol (AMPD), more preferably AMP. These agents, which are widely used in the hair spray industry, produce neutralized copolymers having good hydrocarbon tolerance and shampoo removability.

For the purposes of the invention, the neutralization is preferably as complete as practical. Generally, at least 70%, preferably 80%, and more preferably 90% or more of the carboxyl groups must be neutralized to realize a high degree of hydrocarbon tolerance. While total (100%) neutralization is possible, care must be taken to avoid over-neutralizing the system as the presence of excess neutralizing agents can affect formulation stability.

## 3.3 HAIR SPRAY FORMULATIONS

In addition to the polymers which are neutralized by the appropriate neutralizing agent as described in Section 3.2, the only other essential ingredients in hair spray formulations are the solvent and the propellant. While in some cases, particularly with chlorofluorocarbons, the propellant can be used as the solvent also, it is anticipated that the materials of this invention will be primarily used with non-halogenated solvents and hydrocarbon propellants. In these formulations, the solvents of choice are alcohols, particularly the low boiling, more volatile alcohols.

In general, $C_1-C_4$ straight and branched-chain alcohols can be used, with ethanol, propanol, and isopropanol being the preferred solvents. In addition to their excellent solubilizing properties, these solvents are quite volatile (and, thus, evaporate quickly) and are compatible with containers ordinarily used for pressurized aerosols.

While the polymers used in these formulations are compatible with virtually any of the aerosol propellants known to those skilled in the art including halocarbons such as trichlorofluoromethane, it is preferred to use non halogenated hydrocarbons as the propellants to avoid the release of halocarbons into the atmosphere. Preferred propellants are the lower boiling hydrocarbons, preferably $C_3$-$C_6$ straight and branched chain hydrocarbons, more preferably propane, butane, isobutane and mixtures thereof. Other propellants suitable for use in these formulations include ethers such as dimethyl ether.

In general, the method for preparing the hair spray formulations of this invention involves dissolving or diluting the copolymer in the selected solvent(s), adding the neutralizing agent, and subsequently adding any optional compounds whose presence may be desired, and combining the resultant solution with the selected aerosol propellant.

It should be noted that the novel hair spray formulations of this invention will, in all cases, contain at least four essential components. The first and second of these components are the active ingredients which comprise one or more of the above-described copolymers, which serve as the fixative for the formulation, and an appropriate neutralizing agent as described in Section 3.2. The third component comprises one or more solvents which serve as vehicles for the binder. The last component is the propellant which serves to effect the discharge of the aforedescribed fixative and vehicle from the container wherein the formulation is packaged. Water is not ordinarily present, but may be included in some formulations, as may other optional ingredients.

With regard to proportions, the final hair spray formulations typically contain the neutralized copolymeric fixative in a concentration ranging from about 0.5 to 9%, by weight; the solvent in a concentration ranging from about 10 to 90%, by weight; and, the propellant concentration ranging from 10 to 85%, by weight, wherein all percentages total 100%. These proportions should, however, be considered as being merely illustrative inasmuch as it may well be desirable to prepare operable formulations having concentrations of components which fall outside the above suggested ranges for particular applications.

As stated supra, optional additives may also be incorporated into the hair fixing formulations of this invention in order to modify certain properties thereof. Among these additives may be included; plasticizers such as glycols, phthalate esters and glycerine; silicones; emollients, lubricants and penetrants such as lanolin compounds; protein hydrolyzates and other protein derivatives; ethylene oxide adducts, and polyoxyethylene cholesterol; U.V. absorbers; dyes and other colorants; and, perfumes. The copolymeric binders of this invention show little or no tendency to chemically interact with such additives.

The resulting hair fixing formulations exhibit all of the characteristics required of such a product. Their films are transparent, glossy, flexible and strong. They possess good antistatic properties, adhere well to hair, are easily removed by soapy water or shampoos. Further, when on the hair the films allow the hair to be readily recombed, do not yellow on aging, do not become tacky when exposed to high humidities, and have excellent curl retention under high humidity conditions.

## 4. EXAMPLES

The following examples further illustrate the preferred embodiments of this invention and are not intended to be illustrative of all embodiments.

## 4.1 PREPARATION OF COPOLYMERS

In a typical method for the polymerization of copolymers used in the aerosol hair fixing formulations of this invention, a reaction vessel equipped with a condenser and a means for mechanical agitation was charged with a monomer mixture consisting of 45 parts (by weight) isobutylmethacrylate, 30 parts (by weight) N-t-octyl acrylamide, 25 parts (by weight) acrylic acid, 2.0 parts (by weight) of a polymerization initiator, and 100 parts (by weight) of ethanol. Initially, 15% of the mixture and initiator were added, the remainder were added slowly over a 3 - 4 hour period with constant agitation.

The contents were then heated to the reflux temperature of the system and held there for a period of 6 hours whereupon an additional 1.0 part of initiator was added thereto. The system was then held at reflux for an additional 4 hours whereafter the reaction was cooled to 30°C and the polymer was recovered by standard separation means. This was retained as Sample No. 3.

5

The other polymers evaluated in the subsequent examples were either obtained from commercial sources or prepared in a similar manner.

## 4.2 PREPARATION OF FORMULATIONS

In a typical preparation, the hair spray formulations were prepared as follows:

1. The solvent was charged to an agitated mixing vessel at 15-20° C;

2. The polymer mixture is then added slowly to assure an even dispersion, and then neutralized to the desired % neutralization by the addition of the neutralizing agent;

3. The mixture is stirred until the neutralized polymer is dissolved and any optional ingredients are then added;

In this way, a wide variety of formulations were prepared as set forth in Table I.

TABLE I

| Summary of Polymer Systems Used to Prepare Hair Spray Formulations | | | | | | | | | | | |
|---|---|---|---|---|---|---|---|---|---|---|---|
| | Sample No./Wt. % of Monomer | | | | | | | | | | |
| Monomer | 1[a] | 2 | 3 | 4 | 5 | 6 | 7 | 8 | 9 | 10 | 11 |
| N-t-octylacrylamide | 30 | 30 | 30 | 30 | 30 | 15 | 50 | 50 | 30 | 75 | 65 |
| isobutylmethacrylate | 51 | 48 | 45 | 40 | 30 | 60 | 31 | -- | 43 | -- | -- |
| acrylic acid | 19 | 22 | 25 | 30 | 40 | 25 | 19 | 20 | 22 | 25 | 25 |
| hydroxypropylmethacrylate | -- | -- | -- | -- | -- | -- | -- | -- | 5 | -- | -- |
| vinyl acetate | -- | -- | -- | -- | -- | -- | -- | 20 | -- | -- | -- |
| methylacrylate | -- | -- | -- | -- | -- | -- | -- | 10 | -- | -- | -- |
| styrene | -- | -- | -- | -- | -- | -- | -- | -- | -- | -- | 10 |
| Intrinsic viscosity[b] | .35 | .35 | .35 | .35 | .33 | .39 | .31 | .23 | .42 | .12 | .25 |
| Notes | | | | | | | | | | | |

[a] a commercially-available formulation

[b] Intrinsic viscosity of each copolymer was measured on an Ostwald-type viscometer

These polymer samples were examined in subsequent examples using anhydrous ethanol as a solvent, and neutralizing 90% of the carboxyl groups with AMP.

## 4.3 HYDROCARBON TOLERANCE

To assess the maximum quantity of hydrocarbon which can be tolerated in an aerosol formulation by the neutralized polymer mixtures, the polymer samples were formulated as 2% (by weight) solids using anhydrous ethanol as the solvent and A-46 propellant, a hydrocarbon propellant comprising approximately 80% (by weight) isobutane and 20% (by weight) propane. The desired degree of neutralization (90%) was achieved using AMP as the neutralizing agent. The propellant was then added to the desired proportion and the formulations, in clear glass tubes, were chilled to -10° C. The maximum amount of propellant tolerated (i.e., that above which phase separation, as evidenced by the onset of turbidity, occurred) at this temperature was observed. The results are presented in Table II:

TABLE II

| Results of Hydrocarbon Tolerance Tests | |
|---|---|
| Polymer Sample No. | Max. Amount of A-46 (in weight %) Tolerated |
| 1 | 60 |
| 2 | 55 |
| 3 | 50 |
| 4 | 50 |
| 5 | 40 |
| 6 | 50 |
| 7 | 60 |
| 8 | 55 |
| 9 | 50 |
| 10 | 60 |
| 11 | 55 |

The above data clearly demonstrates that increasing the acrylic acid content above 19% that of Versacryl 40, Sample 1, results in only a minor decrease in hydrocarbon tolerance; the difference becomes marked at 40% acrylic acid (Sample 5).

Further, varying the proportions of the acrylamide and methacrylate, and substituting different methacrylates or styrene for IBMA (Samples 6-11) still result in good degrees of hydrocarbon tolerance.

## 4.4 SHAMPOO REMOVABILITY

To assess the shampoo removability of the hair spray formulations of this invention, a series of 4% solutions of the 11 copolymers produced in Example 4.2 in anhydrous ethanol were produced. The copolymers were 90% neutralized with AMP and charged to a spray container and examined.

These formulations were each sprayed on 8 swatches of 10″ Brown European Virgin hair, a total of 20 times (10 times each on the front and back); after a 1/2 hour air-drying period, the spraying was repeated and the swatches were air-dried for an additional hour. Immediately thereafter each swatch was wetted with warm water, squeezed-dry, and washed for 30 seconds with three drops of Prell shampoo. The swatches were then rinsed for 30 seconds, squeezed dry, and oven-dried at 120° F. Each sample was then evaluated for stiffness, flake and feel.

Using the above procedure, Samples 2-11 were all found to have significantly less stiffness and flake and softer feel, all indicative of better shampoo removability, than Sample 1. This difference was observed in each of the 8 replicate trials performed on each sample. The results were also analyzed statistically and the differences were found to be significant at a 95% confidence level. Thus, all samples examined exhibited a highly desirable degree of shampoo removability.

To further assess shampoo removability, Samples 1 and 3 were subjected to an additional washing as described above. After the second wash, Sample 3 appeared to be removed completely (as evidenced by lack of flake and non-stiff, soft feel), while Sample 1 still contained polymer residue. Thus, it appears that shampoo removability increases as acrylic and content is increased.

It is apparent that many modifications and variations of this invention as hereinabove set forth may be made without departing from the spirit and scope thereof. The specific embodiments described are given by way of example only and the invention is limited only by the terms of the appended claims.

## Claims

1. A hair fixing composition for use in aerosol compositions which comprises a copolymer of:
   a. from about 0 to about 60% (by weight) of a $C_3$-$C_{12}$ alkyl acrylate or methacrylate;
   b. from about 15 to about 75% (by weight) of a $C_4$ - $C_{10}$ N-substituted acrylamide; and

7

c. from about 20 to about 35% (by weight) of acrylic acid or methacrylic acid, wherein all percentages total 100 and wherein at least 70% of the carboxylic and functionalities in said copolymer are neutralized by an alkaline neutralizing agent.

2. The composition of Claim 1 which further comprises about 2.5 to about 20% (by weight) of one or more comonomers selected from the group consisting of acrylic and methacrylic esters of hydroxyalkyl esters of acrylic and methacrylic acids, $C_1$-$C_4$ alkyl/$C_2$-$C_4$ amino alkyl esters of acrylic and methacrylic acids, styrene and alkyl substituted styrene monomers, vinyl esters, $C_1$-$C_8$ dialkyl maleates, N-vinyl pyrrolidone, and N-substituted alkyl ($C_1$-$C_8$) maleamic acid.

3. The composition of Claim 1 wherein the alkaline neutralizing agent is 2-amino-2-methyl-1-propanol or 2-amino-2-methyl-1,3-propanediol.

4. A hair spray formulation comprising

(a) from about 0.5 to about 9% (by weight of a hair fixing composition which comprises from about 0 to about 60% (by weight) of a $C_3$-$C_{12}$ alkyl acrylate or methacrylate; from about 15 to about 75% (by weight) of a $C_4$-$C_{10}$ N-substituted acrylamide; and from about 20 to about 35% (by weight) of acrylic acid or methacrylic acid, wherein all percentages total 100 and wherein at least 70% of the carboxylic acid functionalities in said copolymer are neutralized by an alkaline neutralizing agent,

(b) from about 10 to about 90% (by weight) of a solvent; and

(c) from about 10 to about 90% (by weight) of a hydrocarbon propellant, wherein all percentages total 100.

5. The formulation of Claim 4, wherein the solvent is a $C_1$-$C_4$ straight or branched chain alcohol.

6. The formulation of Claim 4, wherein the hydrocarbon propellant is selected from group consisting of $C_3$-$C_6$ straight chain hydrocarbons, $C_4$-$C_6$ branched chain hydrocarbons, and mixtures thereof.

7. The formulation of Claim 4, wherein the hair fixing composition further comprises about 2.5 to about 20% (by weight) of one or more comonomers selected from the group consisting of acrylic and methacrylic esters of hydroxyalkyl esters of acrylic and methacrylic acids, $C_1$-$C_4$ alkyl/$C_2$-$C_4$ amino alkyl esters of acrylic and methacrylic acids, styrene and alkyl substituted styrene monomers, vinyl esters, $C_1$-$C_8$ dialkyl maleates, N-vinyl pyrrolidone, and N-substituted alkyl ($C_1$-$C_8$) maleamic acid.

8. The formulation of Claim 4 wherein the alkaline neutralizing agent is 2-amino-2-methyl-1-propanol or 2-amino-2-methyl-1,3-propanediol.